# EUROPEAN PATENT APPLICATION

(11) **EP 0 887 082 A2**
(43) Date of publication of application: **30.12.1998**
(21) Application number: 98304889.3
(22) Date of filing: 22.06.1998
(51) Int. Cl.: A61M 5/32, A61M 5/50

(54) **Shielded needle assembly**

(30) Priority: 23.06.1997 US 880199
(71) Applicant: Becton, Dickinson and Company, Franklin Lakes, New Jersey 07417-1880 (US)
(72) Inventor: Sweeney, Niall, Rutherford, New Jersey 07070 (US); Morigi, Adriano, Rutherford, New Jersey 07070 (US)
(74) Representative: Ruffles, Graham Keith

(57) **Abstract**

A shielded needle assembly has an elongate needle that defines an axis having a pointed distal end, a proximal end and a passageway therethrough. The assembly also has a hub that is mountable on a fluid handling device with an axial opening therethrough to receive the needle with the distal end projecting outwardly therefrom so that the fluid handing device is in fluid communication with the passageway. The hub has an annular weakened area therein that defines a needle portion and a fluid handling device attachment portion. The assembly further includes an elongate shield with a proximal end, a distal end and a sidewall that defines a cavity. The shield has a slot extending from the proximal end to the distal end. The shield is attached to the needle attachment portion of the hub with a hinge that provides for pivotal movement of the shield between an open position where the pointed distal end of the needle is exposed for use and a closed position where at least the pointed distal end of the needle is disposed within the cavity through the slot and is substantially protected from inadvertent exposure. In the assembly of the invention, the needle portion and the fluid handling device attachment portion of the hub are frangibly detachable from each other at the annular weakened area by a force applied to the assembly transverse to the axis and distal to the annular weakened area.

## Description

Field of Invention: The present invention relates to shielded hypodermic needle assemblies and more particularly to a shield assembly that attaches to the hub of the needle and allows use of the needle on a syringe.

Description: In the medical arts, sharp pointed needles are used for a variety of procedures . Devices having sharp pointed needles are used for administering fluids to patients either directly or into intravenous apparatus, and in various blood drawing applications either with syringes or with specialized holders for filling evacuated tubes.

Exposure to blood borne pathogens is a recognized hazard by anyone associated with the medical arts. As a result of this recognition, numerous protocols for use of needles have been developed and are practiced. The problem of transmission of blood borne pathogens not only exists for the physician, nurse or phlebotomist using the needles, but also for support workers all through the hospital. Since most needles in use today are single-use and disposable, hospital service personnel are at risk from needles that are not properly handled by the users.

The use protocols generally dictate in detail when and how a needle will be used and how it should be disposed of. The problem with many protocols for handling needles is that they often require users to perform additional steps in a procedure.With the press of time and simple carelessness certain practices regarding handling of used needles are sometimes disregarded and injuries still occur. The medical device industry has responded to the problem by producing a wide variety of sharps collectors, needle shielding devices and the like to assist practitioners in their need to reduce the occurrence of needle injuries.

Many devices have been developed for shielding needles after use to avoid exposing other workers to used needles. A representative listing of many of these devices is found in United States Patent 4,982,842 to Hollister et al. Hollister et al. lists 90 U.S. patents of various devices for guarding a needle as part of the background for the present shielded needle container. Hollister et al. discloses a stand alone adapter that has male and female ends for mating with a needle assembly and the ejection end of a syringe. The device of Hollister et al. includes a housing mounted to the adapter which may be pivoted to a position in alignment with the needle for enveloping the needle and locking the needle to retain it in the housing. The Hollister et al. device increases the unusable or "dead" volume of the device on which the adapter is mounted, requires an additional part which increases the projection of the needle hub, and the mechanism for holding the cap onto the needle clips onto the needle itself, which may create an aerosol of any fluid remaining on the needle.

United States Patent 5,207,653 to Janjua et al. discloses a needle cap with a longitudinal slit having a width greater than the width of a needle. According to Janjua et al., the needle cap is adapted to be pivotally connected with the needle and hub piece. Janjua et al. also discloses that the needle cap is usable with a syringe or with a needle holder for fluid collection tubes.

Many of the devices listed in the background of the Hollister et al. patent, the Hollister et al. invention itself and the Janjua et al. invention all attempt to address the recognized need to protect medical and service personnel from needle sticks. There are several recurrent problems in varying degrees with all these devices. Many of the devices are somewhat complex, hence are significantly more costly than an unprotected device. Many of the devices increase the complexity or increase the difficulty of performing a procedure. Some devices are so specific that they preclude use of the device in certain procedures. For these and similar reasons most of the devices in the Hollister et al. background have never been successfully commercialized.

An additional problem common to most of the already disclosed shielded devices is that while almost all shielded needle assemblies are intended to be single use, a determined effort by a person intent on misuse of many of these shielded needle devices can often overcome the shielding device and re-expose a fully functional, albeit contaminated, hypodermic needle. Although there already are many shielded needle devices, there is still a need for a shielded needle device that is easily manufactured, applicable to many devices and simple to use. Additionally, the if the shielded needle device included provisions to allow the practitioner to render the hypodermic needle substantially unusable after its proper use substantially without extra manipulations or thought, the art of shielded needles would be advanced. Such a device is described below.

### Summary

A shielded needle assembly of the present invention includes an elongate needle that defines an axis having a pointed distal end, a proximal end and a passageway therethrough. The assembly also has a hub that is mountable on a fluid handling device with an axial opening therethrough to receive the needle with the distal end projecting outwardly therefrom so that the fluid handing device is in fluid communication with the passageway. The hub has an annular weakened area therein that defines a needle attachment portion and a fluid handling device attachment portion. The assembly further includes an elongate shield with a proximal end, a distal end and a sidewall that defines a cavity. The shield has a slot extending from the proximal end to the distal end. The shield is attached to the needle attachment portion of the hub with a hinge that provides for pivotal movement of the shield between an open position where the pointed distal end of the needle is exposed for use and a closed position where at least the pointed distal end of the needle is disposed within the cavity through the slot and is substantially protected from inadvertent exposure. In the assembly of the invention, the needle attachment portion and the fluid handling device attachment portion of the hub are frangibly detachable from each other at the annular weakened area by a force applied to the assembly transverse to the axis and distal to the annular weakened area

The shielded assembly of the invention allows a practitioner to render the device substantially unusable after its authorized use by closing the shield and simply applying additional transverse force to the shield to snap off the needle containing portion of the hub. Once the needle containing portion of the hub is snapped off, the needle assembly is substantially worthless to someone intent on misuse of the hypodermic needle because it is virtually impossible to reattach the hypodermic needle to a syringe. Many other available shielded needle assemblies protect personnel from inadvertent exposure to the pointed distal end of the needle, but, unlike the present invention, generally cannot prevent a determined individual intent on misuse of the discarded device from overcoming their shield mechanisms to provide a fully functional, albeit contaminated, hypodermic needle.

### Brief Description of the Drawings

Fig. 1 is an exploded perspective view of the shielded needle assembly of the invention and a syringe;
Fig. 2 is a perspective view of the assembly of Fig. 1 as mounted on the syringe with the shield in the as-shipped position;
Fig. 3 is a longitudinal cross-sectional view of the assembly of the invention from Fig. 2, taken along the line 3-3;
Fig. 4 is a cross-sectional view of the assembly of the invention, analogous to Fig. 3, with the shield in the open position;
Fig. 5 is a cross-sectional view of the assembly of the invention, analogous to Fig. 3, with the shield closed and latched;
Fig. 6 is a cross-sectional view of the assembly of the invention with the shield closed and latched, analogous to Fig. 5, illustrating the frangible detachment of the assembly at the hub;
Fig. 7 is a vertical cross-sectional view of the assembly of the invention from Fig. 2 taken along the line 7-7; and
Fig. 8 is a vertical cross-sectional view of the assembly of the invention, taken from Fig. 5, along the line 8-8;
Fig. 9 is a cross-sectional view of the shielded needle assembly of Fig. 1 with the shield, hinge and hub formed as a monolithic single unit of manufacture; and
Fig. 10 is a perspective view of an alternate embodiment of the needle assembly of the invention with a mechanical pivotal hinge.

### Detailed Description

While this invention is satisfied by embodiments in many different forms, there is shown in the drawings and herein described in detail, several embodiments of the invention with the understanding that the present disclosure is considered as exemplary of the principles of the present invention and is not intended to limit the scope of the invention to the embodiments illustrated. The scope of the invention is measured by the appended claims and their equivalents.

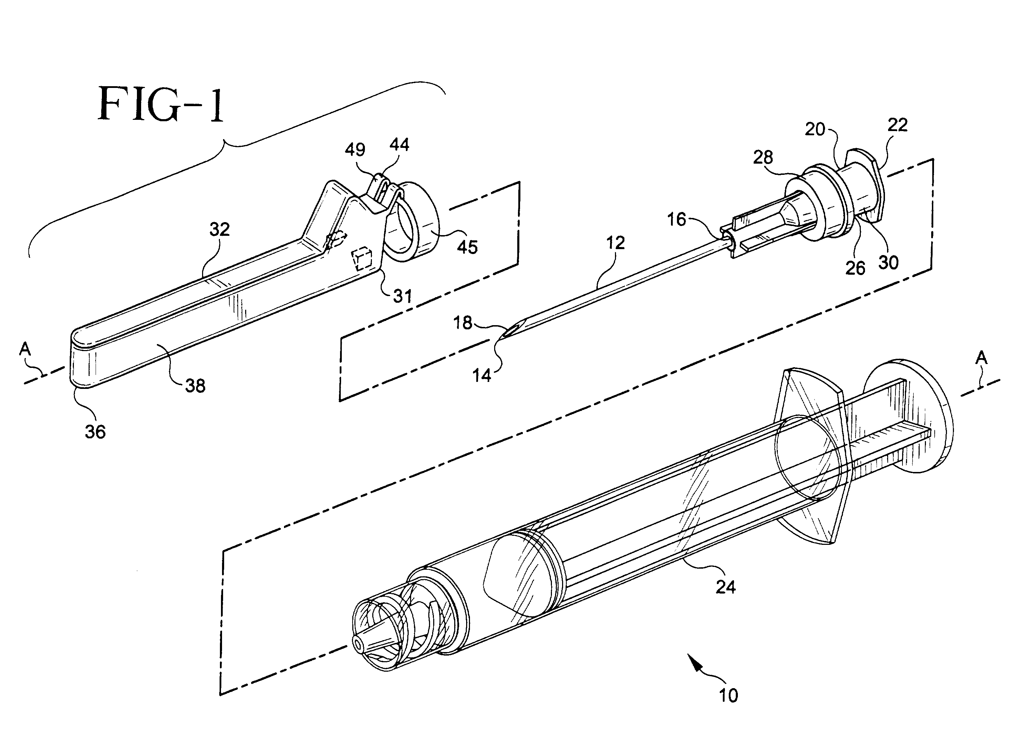

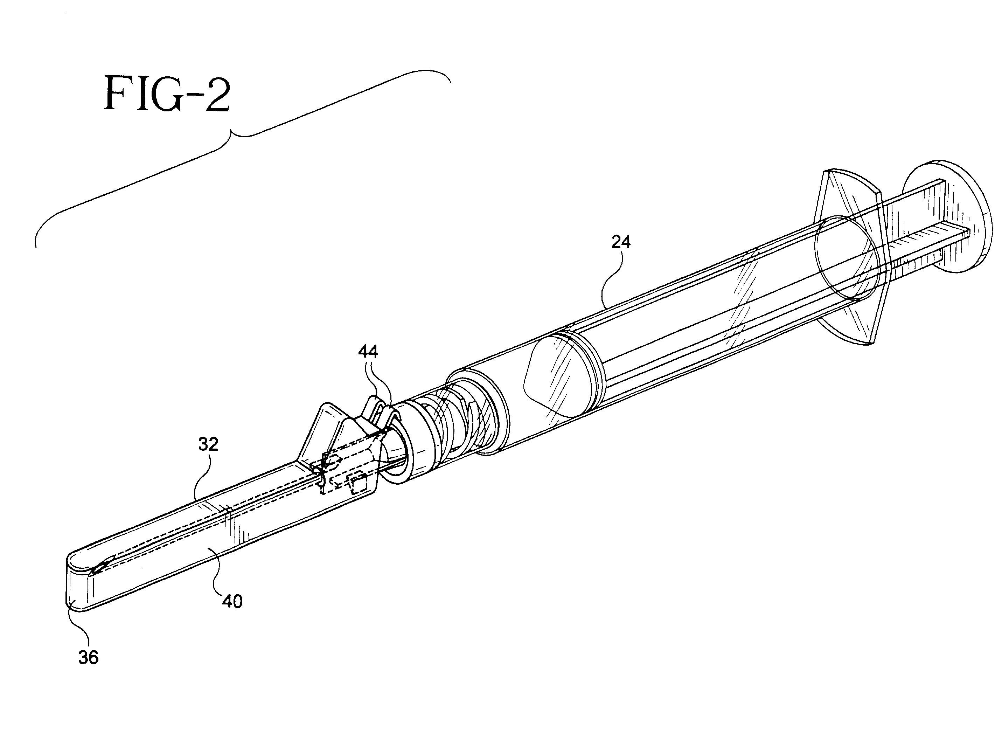

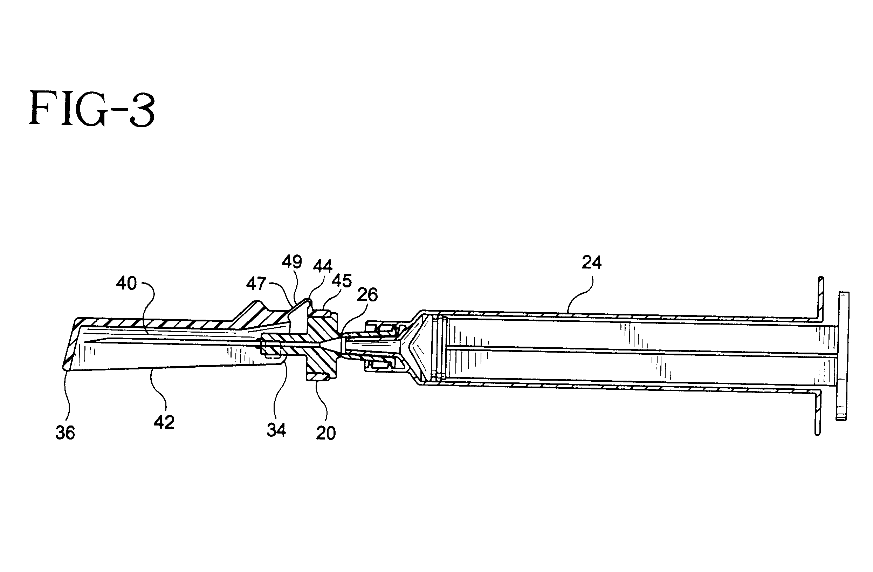

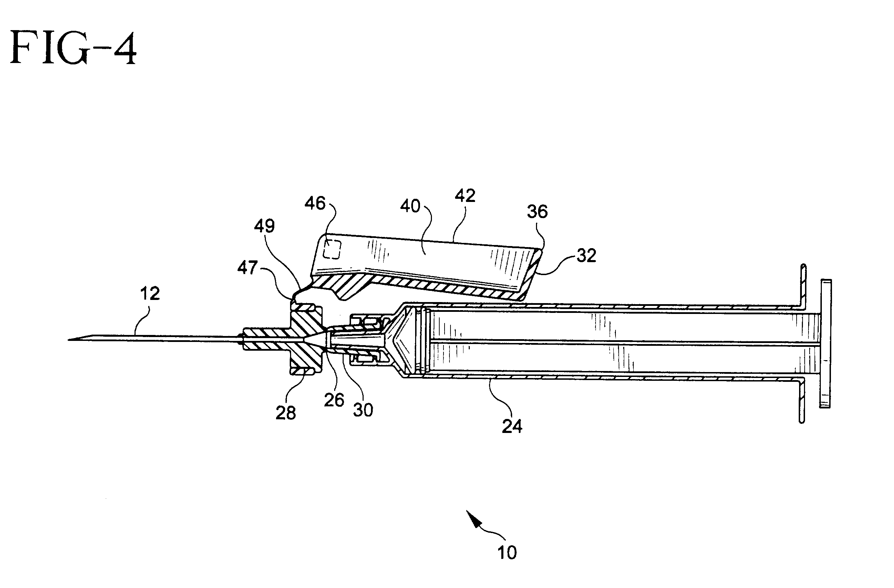

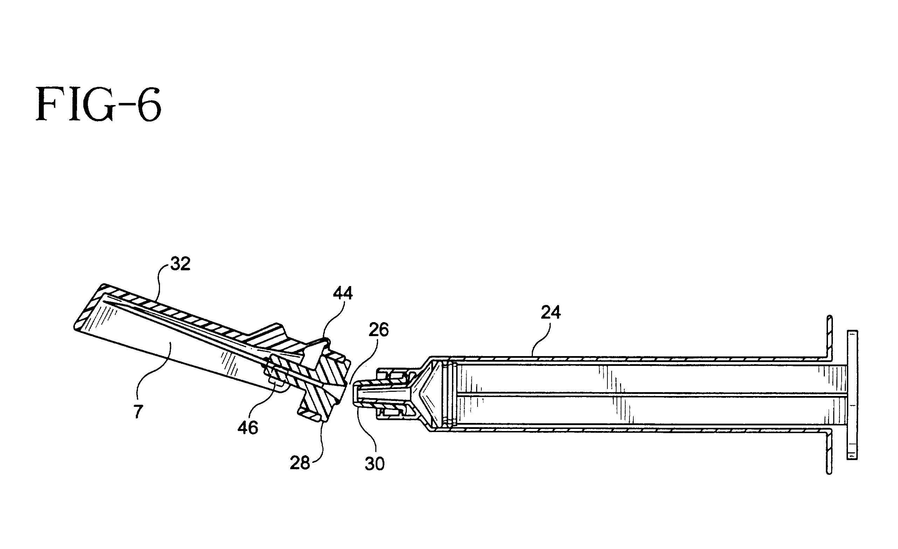

Referring to Figs. 1-8, a shielded needle assembly 10 of the invention includes an elongate needle 12 defining an axis A with a pointed distal end 14, a proximal end 16 and a passageway 18 therethrough. Assembly 10 has a hub 20 with an axial opening 22 therethrough mountable on a fluid handling device such as a syringe 24 to receive needle with said distal end 14 projecting outwardly therefrom so that fluid handing device 24 is in fluid communication with passageway 18 Hub 20 has an annular weakened area 26 therein defining a needle attachment portion 28 and a fluid handling device attachment portion 30. Assembly 10 also includes an elongate shield 32 that has a proximal end 34, a preferably closed distal end 36 and a sidewall 38 that defines a cavity 40. Shield 32 has a slot 42 extending from proximal end 34 to distal end 36. Shield 32 is attached to needle attachment portion 28 of the hub by a hinge 44 to allow pivotal movement between an open position, best seen in Fig. 4, where pointed distal end 14 of the needle is exposed for use and a closed position, best seen in Figs. 2 and 3, where at least pointed distal end 14 of needle 12 is disposed within cavity 40 through slot 42 and is substantially protected from inadvertent exposure. As illustrated in Fig. 6, hub needle attachment portion 28 and hub fluid handling device attachment portion 30 are frangibly detachable from each other at annular weakened area 26 by a force applied to assembly 10 transverse to axis A and distal the annular weakened area.

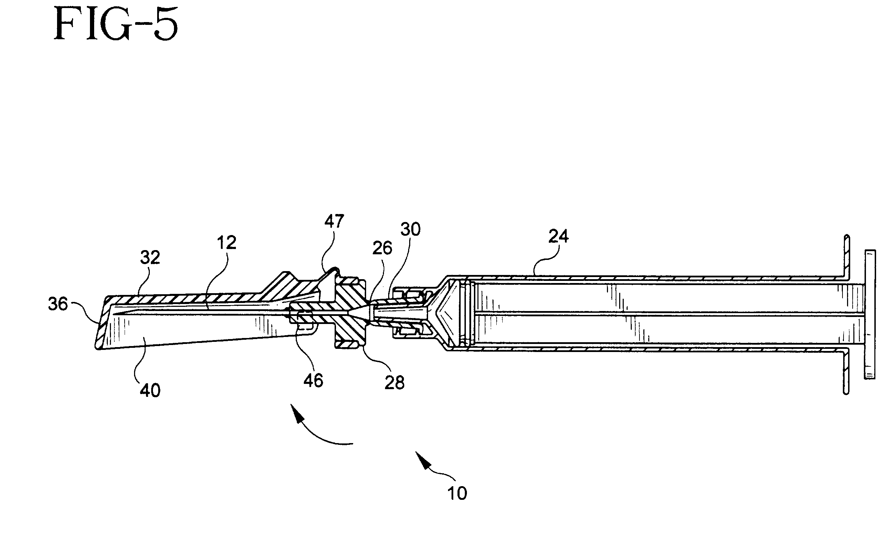

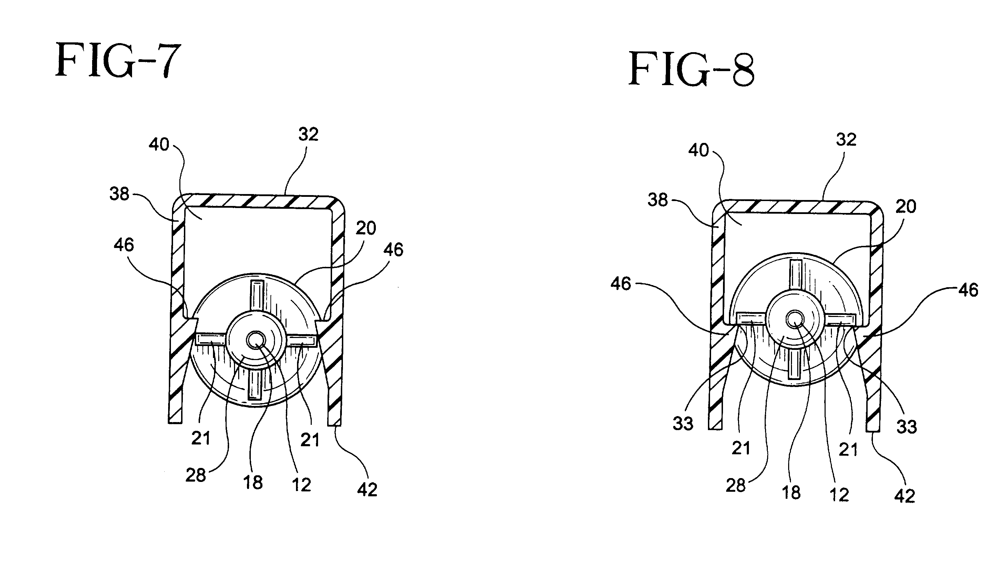

Preferably, shielded needle assembly 10 includes at least one protuberance 46 for engaging hub 20 thereby latching shield 32 to hub 20. Preferably, the pivotal movement about hinge 44 thus further includes a latched position, best seen in Fig. 5, and preferably, the transverse force for frangibly detaching needle attachment portion 28 and fluid handling device attachment portion 30 at annular weakened area 26 is less than a force required to disengage protuberance 46 from hub 20. Figs. 7 and 8 illustrate the interaction between protuberances 46 and engagement portions 21 of the needle hub to form a latch 33 with a release for greater than the force required to frangibly detach needle attachment portion 28 of the hub from fluid attachment portion 30 of the hub.

Preferably, annular weakened area 26 of hub 20 is in the form of a groove 48 or similar reduction in thickness of the hub to facilitate the frangible detachment of the two portions of the hub from each other. The particular shape of the groove may be selected to provide a stress concentration at the desired point of fracture to facilitate the frangible detachment of the two portions from each other. Generally, a sharp corner at the base of the groove provides a focus for applied stress and facilitates the desired fracture.

As shown in Fig. 1, shielded needle assembly 10 hinge 44 may include a mount 45 and needle attachment portion 28 of 20 hub includes an outside surface 21 for receiving mount 45. Suitable ways that mount 45 may be fixedly attached to surface 21 include, but are not limited to, an interference fit, an adhesive bond, a solvent bond, an ultrasonic weld, a thermal weld and the like.

Preferably, fluid device attachment portion 30 of hub 20 includes a female luer fitting 31 for attaching assembly 10 to a fluid handling device such as syringe 24.

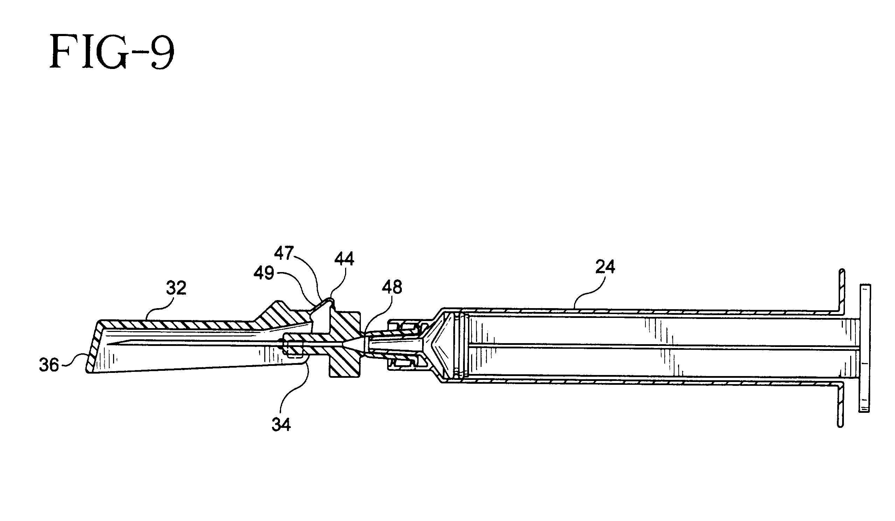

Referring to Fig. 9, shielded needle assembly 10 preferably is monolithically formed as a single article of manufacture from a thermoplastic material and the like by an injection molding process. When assembly 10 is formed in this manner, preferably, hinge 44 is formed as a "living hinge" 47 as part of the injection molding process. A living hinge is formed when a thermoplastic material such as polyethylene, polypropylene, polyvinyl chloride, and the like are formed with a thin, relative to the rest of the article, cross-sectional area 49 at the desired location of the hinge. When the article is formed in the molding tool, the molten thermoplastic material flows through the thin area and, if the article is then subjected to a flexural motion as the article is removed from the molding tool, the molecules are oriented so that the thin area then acts as a hinge.

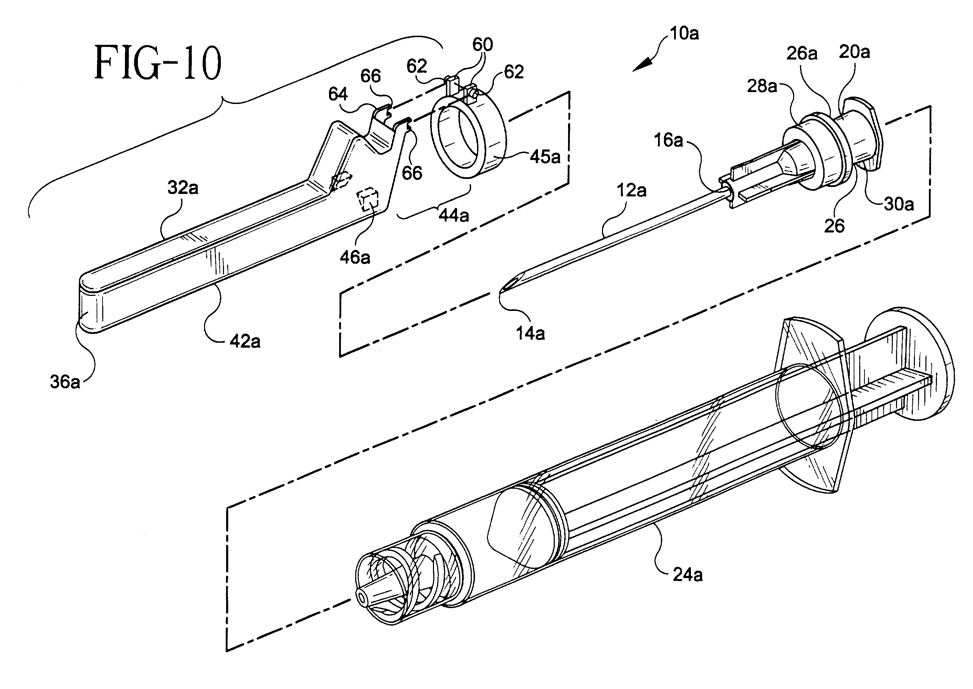

As shown in Fig. 10, an alternate form of hinge 44 is shown, in this embodiment there are elements similar in structure and function to the embodiment of the present invention shown in figs. 1 through 8. Accordingly, substantially similar components that perform substantially similar functions are numbered identically to those components of the embodiment of Figs. 1 through 8 except that a suffix "a" is added to identify those components in Fig. 10. In this embodiment, hub 20a includes two projections 60 with outwardly extending pegs 62 on hub needle attachment portion 28a and shield 32a includes two arms 64 extending proximally on opposite sides of elongate slot 42a, each with arm having an opening 66 therein disposed to engage pegs 62 to form pivotal hinge 44a and attach shield 32a to hub 20a.

The shielded needle assembly of the invention provides practitioners with a simple-to-use reliable way to substantially ensure that there is no secondary unauthorized usage of a needle assembly. The assembly of the invention is simple to manufacture and its usage is intuitive. The practitioner moves the shield to the open position and uses the needle assembly in a normal fashion, the practitioner then closes and latches the shield over the distal point of the needle, and then only needs to apply additional force in the same direction used for latching the shield to cause the frangible detachment of the needle attachment portion of the hub, thereby rendering the needle substantially unavailable for most possible misuse.

## Claims

1. A shielded needle assembly comprising:
an elongate needle defining an axis having a pointed distal end, a proximal end and a passageway therethrough;
a hub having an axial opening therethrough mountable on a fluid handling device to receive said needle with said distal end projecting outwardly therefrom so that said fluid handing device is in fluid communication with said passageway, said hub having an annular weakened area therein defining a needle attachment portion and a fluid handling device attachment portion;
an elongate shield having a proximal end, a distal end and a sidewall defining a cavity, said shield having a slot extending from said proximal end to said distal end,
a hinge for attaching said shield to said needle attachment portion of said hub for pivotal movement between an open position wherein said pointed distal end of said needle is exposed for use and a closed position wherein at least said pointed distal end of said needle is disposed within said cavity through said slot and is substantially protected from inadvertent exposure; and
wherein said needle attachment portion and said fluid handling device attachment portion of said hub are frangibly detachable from each other at said annular weakened area by a force applied to said assembly transverse to said axis and distal to said annular weakened area.

2. The shielded needle assembly of claim 1 wherein said shield further includes at least one protuberance for engaging said hub when said shield is pivotally moved from said open position to a latched position, said thereby latching said shield to said hub, and wherein said pivotal movement further comprises said latched position and wherein said transverse force for frangibly detaching said needle attachment portion and said fluid handling device attachment portion of said hub at said annular weakened area is less than a force required to disengage said protuberance from said hub.

3. The shielded needle assembly of claim 2 wherein said annular weakened area of said hub comprises a groove in said hub.

4. The shielded needle assembly of claim 1 wherein said hub and said shield are monolithically formed as a single article of manufacture.

5. The shielded needle assembly of claim 4 wherein said monolithic formation of said hub and said shield comprises forming said assembly by an injection molding process from a thermoplastic material.

6. The shielded needle assembly of claim 5 wherein said hinge comprises a living hinge.

7. The shielded needle assembly of claim 6 wherein said living hinge comprises an area of reduced thickness, relative to the rest of the shield and hub, said area of reduced thickness having been subjected to a flexural movement as a final step of said injection molding process, thereby forming a living hinge from said area of reduced thickness.

8. The shielded needle assembly of claim 1 wherein said hinge further includes a mount and said needle attachment portion of said hub includes an outside surface for receiving said mount.

9. The shielded needle assembly of claim 8 wherein said mount is fixedly attached to said outside surface of said needle attachment portion by attachment means selected from the group consisting of an interference fit, an adhesive bond, a solvent bond, an ultrasonic weld and a thermal weld.

10. The shielded needle assembly of claim 1 wherein said hub includes two projections with outwardly extending pegs on said needle attachment portion and said shield includes two arms extending proximally on opposite sides of said elongate slot, each arm having an opening therethrough disposed to engage said pegs on said projections thereby forming a pivotal hinge and attaching said shield to said hub.
